# EUROPEAN PATENT APPLICATION

(11) **EP 4 721 815 A1**
(43) Date of publication of application: **08.04.2026**
(21) Application number: 25206482.9
(22) Date of filing: 02.10.2025
(51) Int. Cl.: A61N 5/10

(54) **GANTRY COMPONENTS**

(30) Priority: 02.10.2024 GB 202414482
(71) Applicant: Elekta Limited, Crawley, West Sussex RH10 9BL (GB)
(72) Inventor: CARLANDER, Erik, Crawley, RH10 9BL (GB); EKETJÄLL, Martin, Crawley, RH10 9BL (GB); WIBERG, Kristian, Crawley, RH10 9BL (GB); NYMAN, Markus, Crawley, RH10 9BL (GB)
(74) Representative: Woodhill, Matthew James

(57) **Abstract**

There is provided a radiotherapy system comprising: a rotatable gantry configured to rotate about a rotation axis; an on-gantry component mounted to the gantry, wherein the on-gantry component is configured to travel between at least a first position and a second position on the gantry, and wherein the on-gantry component is mounted to the gantry such that when the gantry is rotated in a first direction about the rotation axis, the on-gantry component is capable of travelling from the first position to the second position under the influence of gravity.

## Description

This disclosure relates to radiotherapy, and in particular systems and methods for initiating and controlling movement of components on a rotating gantry of a radiotherapy system.

### Background

Radiotherapy involves the use of ionising radiation, such as X-rays, to treat a human or animal body. Radiotherapy is commonly used to treat tumours within the body of a patient or subject. In such treatments, ionising radiation is used to irradiate, and thus destroy or damage, cells which form part of the tumour.

A radiotherapy system typically comprises a rotating gantry which supports a beam generation system, or other source of radiation, which is rotatable around a patient. For example, for a linear accelerator (linac) device, the beam generation system may comprise a source of radio frequency energy, a source of electrons, an accelerating waveguide, beam shaping apparatus, etc.

A radiotherapy device will have a rotating gantry drum or a similar structure onto which various components are mounted. As the gantry rotates, the components move around a patient in a circular fashion. Such components may include one or more of: a beam generation system, radiation source, bean shaping apparatus, and so on. Another two exemplary components mounted to the gantry may be the kV and MV imaging systems. The kV imaging system can be used for imaging of the patient and the MV imagine system can be used to verify the radiation delivery system. Both may need to be moved, either to image larger areas or to be moved out of the field of a radiation beam, for example in order to protect sensitive electronics.

Conventional means for moving these imaging systems include linear drives, such as screw or toothed belt drives. Such conventional techniques require multiple moving parts that increase the cost and complexity of the overall radiotherapy system. In addition, the moving parts may need servicing, maintenance, or replacement after a limited lifetime.

The present disclosure seeks to address these and other disadvantages encountered in the prior art.

### Summary

According to a first aspect of the present disclosure, there is provided a radiotherapy system comprising a rotatable gantry configured to rotate about a rotation axis, and an on-gantry component mounted to the gantry. The on-gantry component is configured to travel between at least a first position and a second position on the gantry, and the on-gantry component is mounted to the gantry such that when the gantry is rotated in a first direction about the rotation axis, the on-gantry component is capable of travelling from the first position to the second position under the influence of gravity.

In some embodiments, the radiotherapy system further comprises a first locking mechanism configured to engage and disengage with a portion of the on-gantry component to retain the on-gantry component at the first position. The first locking mechanism prevents the on-gantry component from travelling to the second position when the first locking mechanism is engaged with the on-gantry component.

In some embodiments, the on-gantry component is further capable of travelling from the second position to the first position under the influence of gravity when the gantry is rotated in a second direction about the rotation axis, the second direction being opposite to the first direction.

In some embodiments, the radiotherapy system further comprises a second locking mechanism configured to engage and disengage with a portion of the on-gantry component to retain the on-gantry component at the second position. The second locking mechanism prevents the on-gantry component from travelling to the first position when the second locking mechanism is engaged with the on-gantry component.

In some embodiments, the on-gantry component is configured to move between the first and the second positions without requiring a drive force from a dedicated drive system connected to the on-gantry component.

In some embodiments, the on-gantry component is slidably mounted to one or more rails extending generally between the first and second positions on the gantry, such that a gravitational force acting on the on-gantry component is capable of causing the on-gantry component to slide along the rails. In some embodiments, the on-gantry component is capable of travelling, under the influence of gravity, along the rails, when the gravitational force acting on the on-gantry component is at least partially in the same direction as the direction of travel along the rails.

In some embodiments, the radiotherapy system further comprises a motion control means configured to control the speed of the on-gantry component during travel between the first and second position. The motion control means may comprise a braking system configured to limit the speed of the on-gantry component during at least a portion of the travel between the first and second position. The braking system may comprise a pneumatic braking system comprising a rod located inside and configured to move within a tube, wherein one of the rod and the tube is fixed relative to the on-gantry component and wherein the other one of the rod and the tube is fixed relative to the gantry, such that movement of the on-gantry component between the first and second positions on the gantry causes movement of the rod relative to the tube, and wherein the rod and tube arrangement is configured to apply a braking force to the on-gantry component when the on-gantry component travels between the first and second positions.

In some embodiments, the rod is connected to either the on-gantry component or the gantry via a connecting portion extending through an open end of the tube, and wherein the opposite end of the tube is closed such that movement of the rod within the tube causes expansion and compression of air located in the tube between the rod and the closed end of the tube, and wherein said expansion or compression of air exerts a force on the rod that opposes the movement of the rod relative to the tube. In some embodiments, the closed end of the tube comprises a valve configured to control the flow of air into and out of the tube. In some embodiments, the tube comprises a central portion and end portions located at opposite ends of the central portion, wherein the internal diameter of the central portion is larger than the internal diameter of either end portion. In some embodiments, the pneumatic braking system comprises a first rod configured to move within a first tube, the first rod and tube configured to apply a braking force to the on-gantry component when the on-gantry component travels towards the first position, and a second rod configured to move within a second tube, the second rod and tube configured to apply a braking force to the on-gantry component when the on-gantry component travels towards the second position.

In some embodiments, the braking system comprises a magnetic braking system comprising one or more magnets arranged adjacent to one or more conductive non-magnetic elements, wherein either the one or more magnets or the one or more conductive non-magnetic elements are fixed relative to the on-gantry component, and wherein the other of the one or more magnets and the one or more conductive non-magnetic elements is fixed relative to the gantry, such that movement of the on-gantry component between the first and second positions causes relative movement between the one or more magnets and one or more conductive non-magnetic elements. The one or more magnets may comprise an electromagnet, and the radiotherapy system may further comprise a controller configured to control power supply to the electromagnet.

In some embodiments, the one or more conductive non-magnetic elements comprises an elongate metallic plate with a length that substantially corresponds to the distance between the first and second positions on the gantry. The elongate metallic plate may comprise a central portion and end portions located at either end of the central portion, and the plate may comprise a plurality of holes or slots arranged along the length of the central portion.

In some embodiments, the first and second locking mechanism comprises an electromagnetic latch configured to move between a latched position and an unlatched position to retain the on-gantry component at either the first or second position, wherein the electromagnetic latch comprises an electromagnet and a locking pin attached thereto, a spring that biases the electromagnetic latch towards the latched position, and a magnetic component arranged on an opposite side of the electromagnet to the locking pin.

When in the latched position, the locking pin may be configured to engage a portion of the on-gantry component to retain the on-gantry component at either the first or second position, and the electromagnetic latch may be arranged such that power supplied to the electromagnet causes a magnetic attraction force between the electromagnet and the magnetic component to drive the electromagnetic latch to an unlatched position whereby the locking pin is disengaged from the on-gantry component.

In some embodiments, the radiotherapy system may further comprise a counterbalance system configured offset the change in weight distribution on the gantry when the on-gantry component travels between the first and second position, wherein the counterbalance system comprises a counterbalance weight configured to move in generally the opposite direction to the on-gantry component as the on-gantry component travels between the first and second positions on the gantry. The counterbalance weight may be connected to the on-gantry component via one or more of: a synchronizing belt; a wire; a chain pulley system; gears; and link arm mechanics, such that movement of the counterbalance weight is synchronized with movement of the on-gantry component. In some embodiments, the counterbalance weight comprises a radiation attenuating material.

According to a second aspect of the present disclosure, there is provided a method for displacing an on-gantry component of a radiotherapy system of any preceding claim. When the on-gantry component is retained at the first position on the gantry by a first locking mechanism, the method comprises unlocking the first locking mechanism such that the on-gantry component is free to move towards the second position on the gantry. causing the gantry to rotate in a first direction about the rotation axis such that a gravitational force acting on the on-gantry component causes the on-gantry component to travel from the first position to the second position under the influence of gravity, controlling the speed of the on-gantry component whilst the on-gantry component is travelling from the first position to the second position, and when the on-gantry component reaches the second position, retaining the on-gantry component at the second position using a second locking mechanism such that further rotation of the gantry will not cause further movement of the on-gantry component. Controlling the speed of the on-gantry component comprises using a pneumatic or magnetic braking system to limit the speed of the on-gantry component, or rotating the gantry whilst the on-gantry component is travelling between the first and second position to adjust a gravitational acceleration vector acting on the on-gantry component.

There is also provided a system comprising one or processors and one or more computer-readable media, which is optionally non-transitory, wherein the one or more computer-readable media store instructions that, when executed by the one or more processors, cause the one or more processors to carry out a method according to the second aspect, or any one of the other methods disclosed herein.

There is also provided a computer-readable medium (which is optionally non-transitory) storing instructions that, when executed by one or more processors, cause the one or more processors to carry out the method according to the second aspects, or any one of the other methods disclosed herein.

### Figures

Specific embodiments are now described, by way of example only, with reference to the drawings, in which:
Figure 1 depicts a radiotherapy device or apparatus according to the present disclosure;
Figure 2 depicts a first configuration of a gantry comprising an on-gantry component according to the present disclosure;
Figure 3 depicts a second configuration of a gantry comprising an on-gantry component according to the present disclosure;
Figures 4A and 4B depict a braking system for an on-gantry component according to an embodiment of the present disclosure;
Figure 4C depicts an alternative braking system for an on-gantry component according to an embodiment of the present disclosure;
Figure 4D depicts an alternative braking system for an on-gantry component according to an embodiment of the present disclosure;
Figure 5A is a first view of a magnetic braking system for an on-gantry component according to an embodiment of the present disclosure;
Figure 5B is a second view of a magnetic braking system for an on-gantry component according to an embodiment of the present disclosure;
Figures 5C - 5E are side-on views of the magnetic braking system shown in Figures 5A and 5B;
Figure 6A depicts a first configuration of a locking mechanism for an on-gantry component according to an embodiment of the present disclosure;
Figure 6B depicts a second configuration of a locking mechanism for an on-gantry component according to an embodiment of the present disclosure;
Figure 6C depicts a perspective view of the locking mechanism shown in Figures 6A and 6B;
Figure 7 is a flowchart for a method of displacing an on-gantry component of a radiotherapy system according to the present disclosure;
Figure 8 depicts a counterbalance system for an on-gantry component according to an embodiment of the present disclosure;
Figure 9 illustrates a block diagram of one implementation of a radiotherapy system according to the present disclosure; and
Figure 10 illustrates a computer program product storing instructions according to the present disclosure.

### Detailed Description

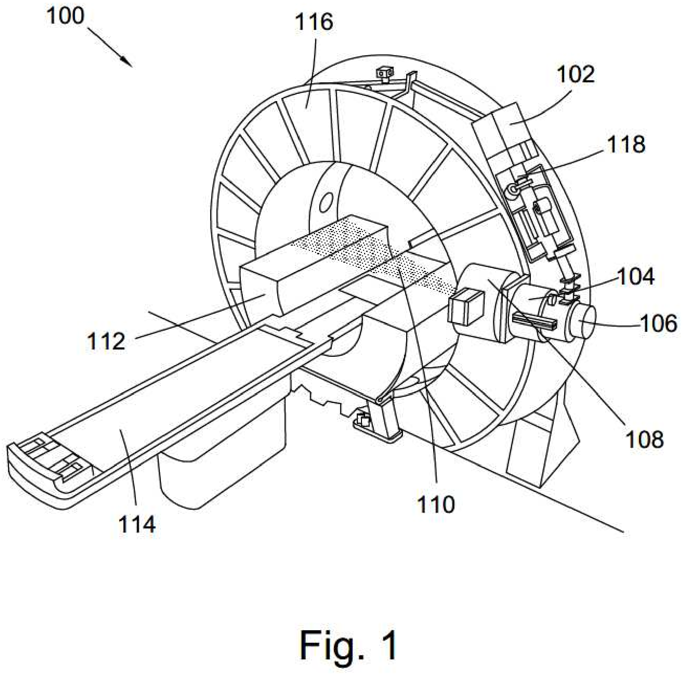

Figure 1 depicts a radiotherapy device suitable for delivering, and configured to deliver, a beam of radiation to a patient during radiotherapy treatment. The device and its constituent components will be described generally for the purpose of providing useful accompanying information for the present invention. The device depicted in figure 1 is in accordance with the present disclosure and is suitable for use with the disclosed systems and apparatuses. While the device in figure 1 is an MR-linac, the implementations of the present disclosure may be any radiotherapy device, for example a linac device.

The device 100 depicted in figure 1 is an MR-linac. The device 100 comprises both MR imaging apparatus 112 and radiotherapy (RT) apparatus which may comprise a linac device. The MR imaging apparatus 112 is shown in cross-section in the diagram. In operation, the MR scanner produces MR images of the patient, and the linac device produces and shapes a beam of radiation and directs it toward a target region within a patient's body in accordance with a radiotherapy treatment plan. The depicted device does not have the usual 'housing' which would cover the MR imaging apparatus 112 and RT apparatus in a commercial setting such as a hospital.

The MR-linac device depicted in figure 1 comprises a source of radiofrequency waves 102, a waveguide 104, a source of electrons 106, a source of radiation 106, a collimator 108 such as a multi-leaf collimator configured to collimate and shape the beam, MR imaging apparatus 112, and a patient support surface 114. In use, the device would also comprise a housing (not shown) which, together with the ring-shaped gantry, defines a bore. The moveable support surface 114 can be used to move a patient, or other subject, into the bore when an MR scan and/or when radiotherapy is to commence. The MR imaging apparatus 112, RT apparatus, and a subject support surface actuator are communicatively coupled to a controller or processor. The controller is also communicatively coupled to a memory device comprising computer-executable instructions which may be executed by the controller.

The RT apparatus comprises a source of radiation and a radiation detector (not shown). Typically, the radiation detector is positioned diametrically opposed to the radiation source. The radiation detector is suitable for, and configured to, produce radiation intensity data. In particular, the radiation detector is positioned and configured to detect the intensity of radiation which has passed through the subject. The radiation detector may also be described as radiation detecting means, and may form part of a portal imaging system.

The radiation source may comprise a beam generation system. For a linac, the beam generation system may comprise a source of RF energy 102, an electron gun 106, and a waveguide 104. The radiation source is attached to the rotatable gantry 116 so as to rotate with the gantry 116. In this way, the radiation source is rotatable around the patient so that the treatment beam 110 can be applied from different angles around the gantry 116. In a preferred implementation, the gantry is continuously rotatable. In other words, the gantry can be rotated by 360 degrees around the patient, and in fact can continue to be rotated past 360 degrees. The gantry may be ring-shaped. In other words, the gantry may be a ring-gantry.

The source 102 of radiofrequency waves, such as a magnetron, is configured to produce radiofrequency waves. The source 102 of radiofrequency waves is coupled to the waveguide 104 via circulator 118, and is configured to pulse radiofrequency waves into the waveguide 104. Radiofrequency waves may pass from the source 102 of radiofrequency waves through an RF input window and into an RF input connecting pipe or tube. A source of electrons 106, such as an electron gun, is also coupled to the waveguide 104 and is configured to inject electrons into the waveguide 104. In the electron gun 106, electrons are thermionically emitted from a cathode filament as the filament is heated. The temperature of the filament controls the number of electrons injected. The injection of electrons into the waveguide 104 is synchronised with the pumping of the radiofrequency waves into the waveguide 104. The design and operation of the radiofrequency wave source 102, electron source and the waveguide 104 is such that the radiofrequency waves accelerate the electrons to very high energies as the electrons propagate through the waveguide 104.

The design of the waveguide 104 depends on whether the linac accelerates the electrons using a standing wave or travelling wave, though the waveguide typically comprises a series of cells or cavities, each cavity connected by a hole or 'iris' through which the electron beam may pass. The cavities are coupled in order that a suitable electric field pattern is produced which accelerates electrons propagating through the waveguide 104. As the electrons are accelerated in the waveguide 104, the electron beam path is controlled by a suitable arrangement of steering magnets, or steering coils, which surround the waveguide 104. The arrangement of steering magnets may comprise, for example, two sets of quadrupole magnets.

Once the electrons have been accelerated, they may pass into a flight tube. The flight tube may be connected to the waveguide by a connecting tube. This connecting tube or connecting structure may be called a drift tube. The electrons travel toward a heavy metal target which may comprise, for example, tungsten. Whilst the electrons travel through the flight tube, an arrangement of focusing magnets act to direct and focus the beam on the target.

To ensure that propagation of the electrons is not impeded as the electron beam travels toward the target, the waveguide 104 is evacuated using a vacuum system comprising a vacuum pump or an arrangement of vacuum pumps. The pump system is capable of producing ultra-high vacuum (UHV) conditions in the waveguide 104 and in the flight tube. The vacuum system also ensures UHV conditions in the electron gun. Electrons can be accelerated to speeds approaching the speed of light in the evacuated waveguide 104.

The source of radiation is configured to direct a beam 110 of therapeutic radiation toward a patient positioned on the patient support surface 114. The source of radiation may comprise a heavy metal target toward which the high energy electrons exiting the waveguide are directed. When the electrons strike the target, X-rays are produced in a variety of directions. A primary collimator may block X-rays travelling in certain directions and pass only forward travelling X-rays to produce a treatment beam 110. The X-rays may be filtered and may pass through one or more ion chambers for dose measuring. The beam can be shaped in various ways by beam-shaping apparatus, for example by using a multi-leaf collimator 108, before it passes into the patient as part of radiotherapy treatment.

The source of radiation may be configured to direct a beam 110 of therapeutic radiation toward a patient in both a coplanar configuration, (where the beam of radiation is generally normal to the rotation axis of the rotatable gantry 116), and a non-coplanar configuration (where the beam of radiation is generally directed at an oblique angle relative to the rotation axis of the rotatable gantry 116). To switch between the coplanar and non-coplanar configurations, the radiation treatment head (including at least the collimator 108) may move between a coplanar configuration as depicted in Figure 1 to a tilted configuration, where the radiation treatment head is no longer generally in the same rotation plane as the gantry and is instead extended array from the gantry in an axial direction (i.e. parallel to the rotation axis of the gantry 116).

In some implementations, the source of radiation is configured to emit either an X-ray beam or an electron particle beam. Such implementations allow the device to provide electron beam therapy, i.e. a type of external beam therapy where electrons, rather than X-rays, are directed toward the target region. It is possible to 'swap' between a first mode in which X-rays are emitted and a second mode in which electrons are emitted by adjusting the components of the linac. In essence, it is possible to swap between the first and second mode by moving the heavy metal target in or out of the electron beam path and replacing it with a so-called 'electron window'. The electron window is substantially transparent to electrons and allows electrons to exit the flight tube.

The subject or patient support surface 114 is configured to move between a first position substantially outside the bore, and a second position substantially inside the bore. In the first position, a patient or subject can mount the patient support surface 114. The support surface 114, and patient, can then be moved inside the bore, to the second position, in order for the patient to be imaged by the MR imaging apparatus 112 and/or imaged or treated using the RT apparatus. The movement of the patient support surface is effected and controlled by a subject support surface actuator, which may be described as an actuation mechanism. The actuation mechanism is configured to move the subject support surface in a direction parallel to, and defined by, the central axis of the bore. The terms subject and patient are used interchangeably herein such that the subject support surface can also be described as a patient support surface. The subject support surface may also be referred to as a moveable or adjustable couch or table. More generally, the patient support surface 114 may be moveable in up to 6 degrees of freedom, for example in a direction parallel to the rotation axis or in a direction perpendicular to the rotation axis. The patient support surface may also be rotatable about one or more axes.

Although not shown in Figure 1, the radiotherapy device depicted in Figure 1 further comprises one or more imaging panels (e.g. KV or MV imaging panel) mounted to the gantry. The imaging panel may be mounted to the gantry by a drive mechanism that is configured to move the imaging panel between two different positions on the circumference of the gantry. The drive mechanism of conventional radiotherapy devices may include a toothed belt drive, screw drive, or any other suitable mechanism driven by a motor that produces linear motion. The movement of the imaging panel may be generally in a tangential direction to the gantry. In other words, the movement between the two positions may be generally orthogonal to a radial direction from the centre of rotation of the gantry.

The radiotherapy apparatus / device depicted in figure 1 also comprises MR imaging apparatus 112. The MR imaging apparatus 112 is configured to obtain images of a subject positioned, i.e. located, on the subject support surface 114. The MR imaging apparatus 112 may also be referred to as the MR imager. The MR imaging apparatus 112 may be a conventional MR imaging apparatus operating in a known manner to obtain MR data, for example MR images. The skilled person will appreciate that such a MR imaging apparatus 112 may comprise a primary magnet, one or more gradient coils, one or more receive coils, and an RF pulse applicator. The operation of the MR imaging apparatus is controlled by the controller.

The controller is a computer, processor, or other processing apparatus. The controller may be formed by several discrete processors; for example, the controller may comprise an MR imaging apparatus processor, which controls the MR imaging apparatus 110; an RT apparatus processor, which controls the operation of the RT apparatus; and a subject support surface processor which controls the operation and actuation of the subject support surface. The controller is communicatively coupled to a memory, e.g. a computer readable medium.

The linac device also comprises several other components and systems as will be understood by the skilled person. For example, in order to ensure the linac does not leak radiation, appropriate shielding is also provided.

### Acceleration drive motion

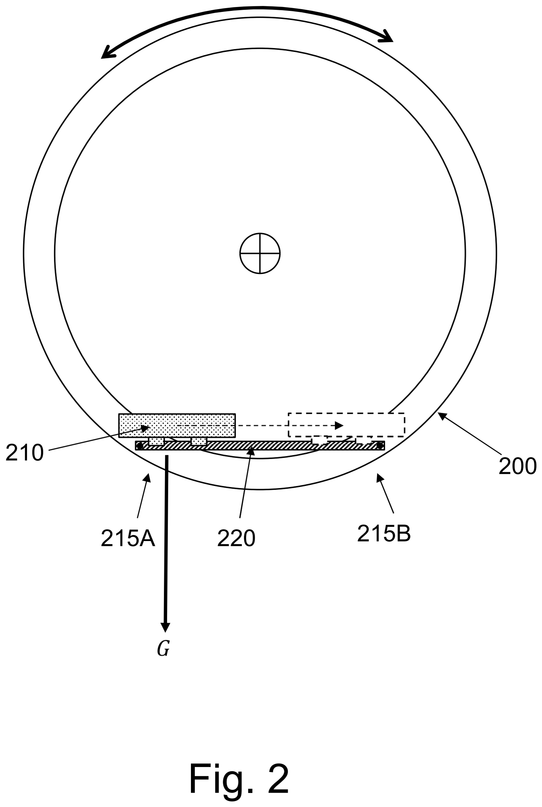

With reference to Figure 2, a schematic image of a ring-shaped gantry 200 of a radiotherapy system according to the present disclosure is shown. The gantry 200 is centred on a rotation axis (indicated by the crossed circle in the centre of the gantry), and the perspective of the Figure is along the direction of the rotation axis. Gantry 200 may be a part of a radiotherapy system, such as system 100, as described above with respect to Figure 1. In general, the gantry may carry many components of the radiotherapy system, such as a radiation source and beam shaping apparatus. For simplicity, these components have been omitted from Figure 2, although the skilled person would appreciate that gantry 200 may be used in any suitable radiotherapy system and may carry any such typical components of the system.

In the embodiment show, the gantry 200 comprises an on-gantry component 210 which is mounted to the gantry 200 in a manner described in more detail below. The on-gantry component 210 may be an imaging panel or detector, such as a kV imaging panel or an MV imaging panel as known to the skilled person. The mon-gantry component 210 will be described hereinafter as an imaging panel 210 (encompassing kV, V or any other type of imaging panel suitable for use with a radiotherapy system), although it would be appreciated by the skilled person that the on-gantry component may in other examples be a different type of component of the radiotherapy system. Whilst examples of the on-gantry component 210 described below relate to imaging panels or detectors, it would be appreciated that the present disclosure may relate more generally to any component mountable to a rotating gantry that may be required to move between two different circumferential positions on the gantry. For example, the on-gantry component may be a radiation source, beam shaping apparatus, or any suitable gantry-mounted component of a radiotherapy system.

In the embodiment shown in Figure 2, the imaging panel 210 is slidably mounted to a rail system 220 which is in turn rigidly fixed to the gantry 200. In the embodiment shown, the rail system comprises one or more parallel rails that are mounted to the gantry in such a way that they are aligned in a direction that is generally perpendicular to a radial direction. More generally, the rail system comprises one or more parallel rails that extend between two different locations of the circumference of the gantry 200. The two different locations are marked as 215A and 215B in Figure 2. The imaging panel is therefore capable of travelling along the rails of the rail system 220 between the two different circumferential positions 215A and 215B of the gantry. Figure 2 shows the imaging panel 210 at the first position 215A and further shows a dashed outline of the panel at the second position 215B on the rail. Whilst the rails depicted in Figure 2 are straight, in other embodiments the rails may be curved, i.e., arcuate, and in some embodiments may generally follow the circumference of the gantry.

As described above, the panel 210 is slidably mounted to the rails of the rail system 220 such that the panel can travel along the rails and between the first position 215A and second position 215B. In other words, the panel is configured to freely travel between the first and second positions without the need for a dedicated drive system. Instead, the panel is configured to slide along the rails under the influence of gravity as the gantry rotates. In more detail, Figure 2 shows the imaging panel 210 located at position 210A (the "left" position in the perspective shown in the Figure). In this position, a gravity acceleration vector (represented by arrow "G") acting on the imaging panel 210 is orthogonal to the rail system 220, meaning that the component of the gravity acceleration vector acting in the direction of the rail is zero. However, when the gantry 200 rotates in a clockwise direction (from the viewpoint of the perspective of Figure 2), the angle between the rail and the horizontal plane increases, and so the component of the gravity acceleration vector acting in the direction of the rail increases. At a threshold angle of gantry rotation in the clockwise direction (corresponding to a threshold angle of the rails to the horizontal place), the force vector component acting in the direction of the rail due to gravity overcomes resistive forces (e.g. friction of the imaging panel sliding along the rail system 220). As a result, the imaging panel travels along the rail from first position 215A to second position 215B. In other words, the imaging panel travels between a first position 215A and second position 215B under the influence of gravity as the gantry rotates.

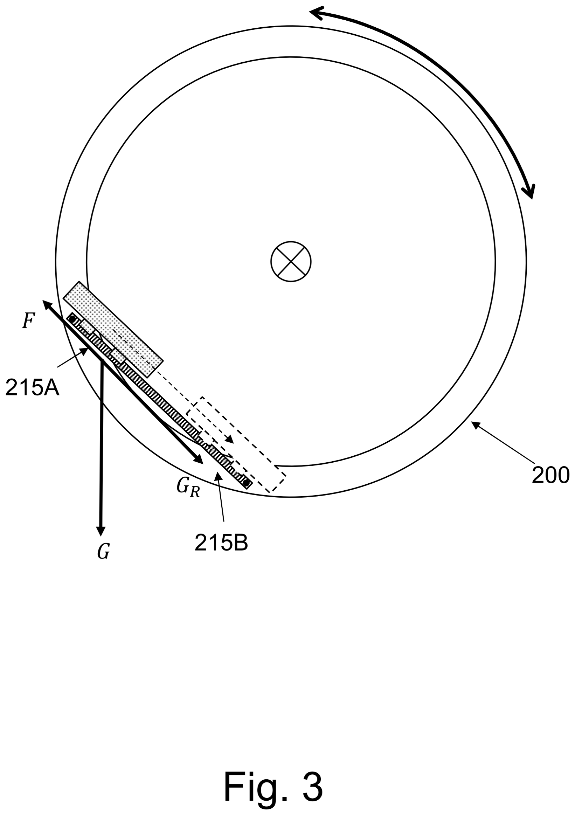

Figure 3 depicts a perspective view of gantry 200 and imaging panel 210 in a rotated arrangement (i.e. where the gantry has been rotated by an acute angle in a clockwise direction relative to the arrangement shown in Figure 2). In this figure, the force arrows representing gravity (arrow G), the component of gravity acting in the movement direction (the direction along the rails) (arrow *G_{R}*), and the frictional forces (arrow F) are shown. These force arrows are shown to demonstrate that, if the gantry rotation angle is sufficient, the component *G_{R}* of the gravitational force acting in the direction of movement of the imaging panel is greater than the opposing frictional forces F and thus the imaging panel movements from the first position 215A to the second position 215B under the influence of gravity. Other forces may also be acting on the imaging panel, however these are not shown or discussed here for simplicity as they are not important for describing movement of the panel between the first and second positions.

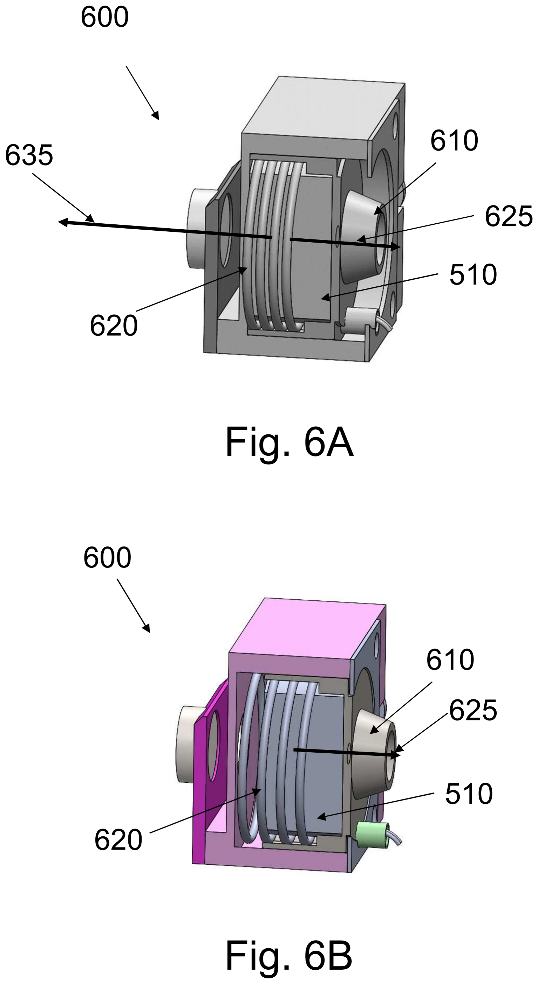

When the imaging panel reaches the second position 215B, a latch or other locking mechanism may be used to fix the imaging panel in place at position 215B. For example, a manual or automated locking pin (e.g. a solenoid driven system) may be used to lock the imaging panel in place in the second position 215B. An exemplary magnetic locking system is described below in relation to Figures 6A to 6C, however the skilled person would be aware of other types of mechanism suitable for securing the imaging panel in position.

Whilst movement from the first position 215A to the second position 215B is described above, it would be appreciated that a corresponding process may be used to move the imaging panel 210 in the opposite direction, i.e. from the second position 215B to the first position 215A. In particular, starting with the imaging panel being located at the second position 215B, the gantry may be rotated in a counter-clockwise direction (from the perspective of either of Figures 2 or 3) to a position at which the rails make an opposite angle to the horizontal plane, such that the component of the gravity acceleration vector *G_{R}* acting in the opposite direction along the rail overcomes the resistive forces *F* and the imaging panel travels along the rail under the influence of gravity to the first position 215A. Once the imaging panel reaches the first position 215A, it may be fixed in place using a latch or locking mechanism as described above.

In general, the imaging panel is capable of travelling under the influence of gravity between the first and second positions, or vice versa, any number of times. The movement of the imaging panel is caused by the rotation of the gantry which leads to the constant gravitational force acting on the imaging panel to align at least partially with the direction in which the imaging panel has freedom of movement (i.e., along the rails of the rail system 220). As such, the imaging panel may be moved along the rail system any number of times, requiring only rotation of the gantry to facilitate the movement. The present disclosure therefore eliminates the need for a dedicated drive system for moving the imaging panel between different circumferential positions, thereby reducing overall design cost and complexity. In effect, by allowing the imaging panel to move freely under the influence of gravity as the gantry rotates, the present disclosure leverages existing gantry rotation to additionally position the imaging panel as necessary. In other words, gantry rotation will already occur during execution of any typical radiotherapy treatment plan. Leveraging this rotation for an additional purpose can therefore reduce overall power consumption associated with moving the imaging panel compared to conventional techniques that rely on a separate powered drive system for drive movement of the imaging panel.

### Motion control

As described above, the on-gantry component (e.g. imaging panel) is slidably mounted to the gantry such that the on-gantry component can freely slide along one or more rails between two different circumferential positions on the gantry. The movement may be driven by rotation of the gantry, allowing the influence of gravity to cause the on-gantry component to slide in a downwards direction from one end of the rails to the opposite end.

In some embodiments, it is desirable to control the speed of movement of the component as it slides along the rails. In some embodiments, this may involve adjusting the gantry angle whilst the component is travelling along the rails to control the speed and/or acceleration of the component. In one example, once the component starts moving along the rails (when the gravitational forces acting in the direction of the rails overcome resistive forces), the gantry may be rotated in an opposite direction to reduce the angle between the rails and the horizontal, thereby reducing the acceleration vector to control the speed of descent of the component. Alternatively, the gantry angle may remain constant whilst the component is moving. In some embodiments, the speed of movement may be controlled using braking means or other speed control means. In some embodiments, the gantry may comprise mechanical end stops located at each end position 215A and 215B. The end stops may comprise buffers such as springs, pneumatic or hydraulic piston buffers, magnets, or the like, that are configured to bring the moving on-gantry component to a stop when the on-gantry component reaches either end position.

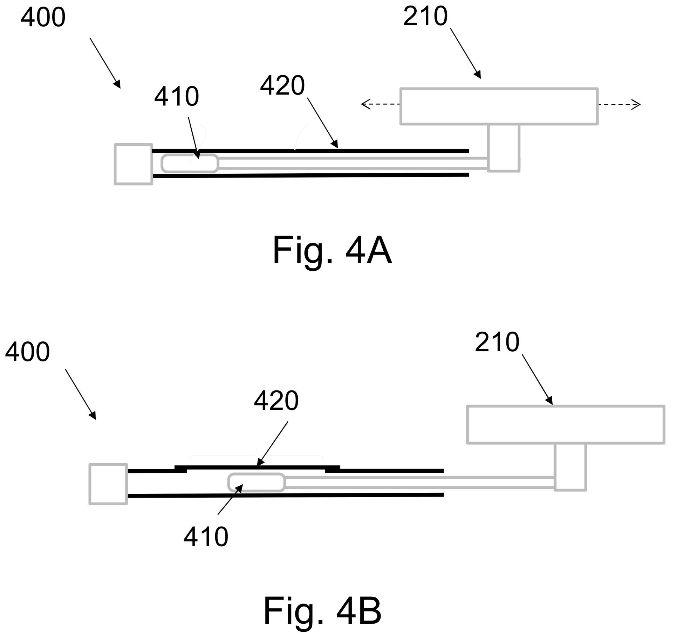

It may also be preferable to reduce the speed of the component as it approaches and reaches each end stop, to reduce the impact energy imparted to the end stop and reduce the risk of damage to any part of the gantry or on-gantry component. Various solutions for controlling the speed of the on-gantry component as it moves between circumferential positions are described below with reference to Figures 4a - 7d.

### Pneumatic speed control

With reference to Figure 4a, a first motion control means comprises a pneumatic braking system 400. The pneumatic braking system 400 comprises a rod 410 located inside a tube 420. The tube 420 is sealed at one end and is rigidly fixed to the gantry. The other end of the tube 420 is open and receives the rod 410 therein. The rod 410 is rigidly fixed to the on-gantry component (e.g. imaging panel 210) and can therefore move relative to the tube as the imaging panel 210 moves along the rail system 220. The relative arrangement of the rod 410 and tube 420 is such that as the imaging panel slides along the rails, the rod moves up and down the tube. For example, as the imaging panel 210 moves from the first position 215A to the second position 215B, the rod travels further into the tube (i.e. towards the closed end). Air in the tube between the rod and the sealed end is therefore compressed. The increased pressure of the air in the tube therefore imparts a pushing force on the rod that opposes the motion of the rod into the tube and thereby opposes the motion of the imaging panel as it moves towards the second position 215B. In the opposite direction, e.g. as the imaging panel moves from the second position 215B back to the first position 215B, the rod travels out of the tube (i.e. away from the sealed end and towards the open end). Air in the tube between the rod and the sealed end is therefore expanded. The reduced pressure of the air therefore imparts a pulling force on the rod that opposes the motion of the rod out of the tube and thereby opposes the motion of the imaging panel as it moves towards the first position 215A.. In this way, the pneumatic braking system 400 is capable of limiting or reducing the speed of the imaging panel as it travels between the two different positions on the circumference of the gantry.

In some embodiments, the diameter and the rod 410 and tube 420 are sized such that the internal diameter of the tube is larger than the diameter of the rod. This allows for a controlled gap between the rod and tube through which air in the tube can escape as the rod travels further into the tube. The difference in size between the diameters of the rod and the tube therefore can be used to determine the speed at which the imaging panel can move. For example, a larger difference in size between the diameters allows for a larger volume of air to escape, providing less resistive force and allowing the imaging panel to travel at a higher speed. Conversely, a smaller difference in size between the diameters allows for a smaller volume of air to escape, providing more resistive force and causing the imaging panel to travel at a lower speed.

In the embodiment depicted in Figure 4a, the diameter of the tube 420 is constant, providing a constant resistance and speed for the full extent of the travel of the imaging panel. In another embodiment depicted in Figure 4b, the diameter of the tube may vary along the length of the tube such that the speed of the imaging panel changes accordingly along its range of travel. Figure 4b shows a tube 420 comprising a wider middle section which allows for a greater speed of the imaging panel during a central stretch of the range of travel. In other words, the internal diameter of the tube 420 is greater in a central portion of the tube compared to the internal diameter at one or both ends of the tube. The central portion with the larger internal diameter may extend between approximately 10% and 90% of the length of the tube, or between approximately 40% and 60% of the length of the tube, or any suitable amount therebetween, e.g. between 25% and 75% of the length of the tube.

In operation of the embodiment depicted in Figure 4b, the imaging panel to which the rod is fixed will be able to travel at a higher speed during a central portion of the range of travel, the central portion corresponding to the central portion of the tube 420 with the larger diameter. This is because, as described above, a greater difference in diameters leads to a larger gap for air to pass through, resulting in less resistive force and a higher movement speed. When the imaging panel approaches either end position (215A or 215B), the rod reaches either end of the tube where the relatively smaller diameter limits air flow, thereby increasing resistance and reducing the movement speed of the rod and in turn the imaging panel fixed to the rod. The embodiments shown in Figures 4A and 4B may be preferable since they employ a simple design with few parts. In addition, the design of the pneumatic braking systems shown in Figure 4A and 4B are passive, meaning that they do not require any power supply in order to effect the braking. The passive and simple design results in a low cost and reduced maintenance requirements.

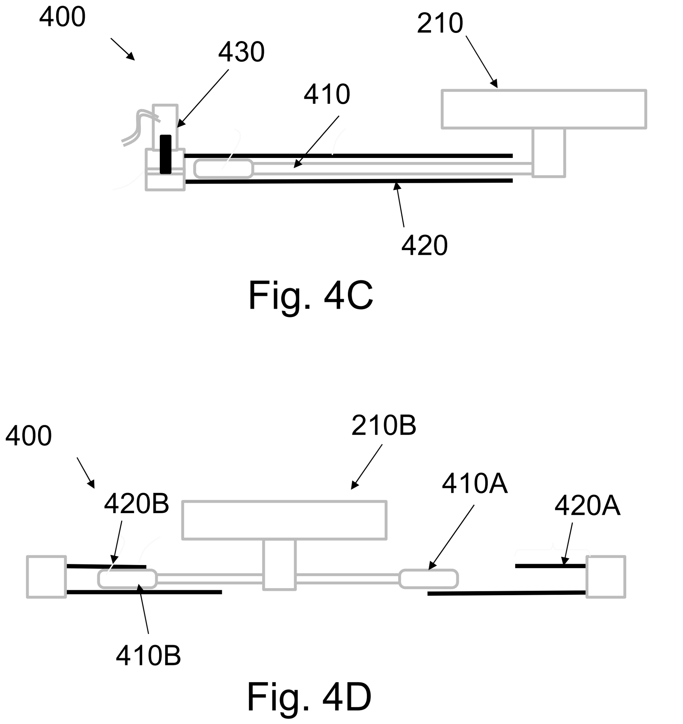

In another alternative embodiment of the pneumatic braking system 400 as shown in Figure 4C, the tube 420 comprises a valve 430 located at the sealed end (the left-hand side as shown in the figure) of the tube 420. The valve may be an electronically controlled valve configured to allow the flow of air into or out of the tube. The amount of air flow can be controlled whilst the imaging panel 210 is moving (and thus whilst the rod 410 is moving within the tube 420) to control the speed of the rod and thus the speed of the imaging panel. For example, as the imaging panel starts moving from the first positions 215A to the second position 215B, the valve may be opened to allow air to flow out of the tube as the rod moves towards the valve. As the imaging panel approaches the second position 215B, the valve may close or prevent further airflow, or the valve may otherwise limit airflow out of the tube, thereby causing a braking effect as described above in relation to Figure 4A. An analogous process would occur for movement of the imaging panel from the second position 215B back to the first position 215A, as the rod moves away from the valve.

In the embodiment depicted in Figure 4C, the internal diameter of the tube 420 may substantially correspond to the diameter of the rod 410. In other words, unlike the embodiments described above in relation to Figure 4A and 4B, the tube and rod may be sized so as to prevent air from escaping through a gap between the two components, in other words forming an airtight seal so that air can only enter or exit the tube via the valve 430.

Referring to Figure 4d, yet another alternative embodiment of a pneumatic braking system is shown, comprising two tubes 420A and 420B and two respective rods 420A and 420B. Each rod-and-tube arrangement may be similar or identical to any of the arrangements described above in relation to Figures 4A - 4C. In this embodiment, a first rod and tube arrangement 410A, 420A is configured to exert a braking force on the imaging panel when the imaging panel approaches the second position 215B from the first position 215A. In more detail, as the imaging panel 210 moves from the first position 215A to the second position 215B, the rod 410A travels further into the tube 420A, which may be sealed or may comprise a valve as described above. The air in the tube 420A is compressed by the rod 410, thereby exerting a braking force on the rod and in turn on the imaging panel. Similarly, the second rod and tube arrangement 410B, 420B is configured to exert a braking force in the same manner on the imaging panel when the imaging panel approaches the first position 215A from the second position 215B.

Whilst the above embodiments have been described in relation to the rod being fixed to the on-gantry component (the moving part) and the tube being fixed to the gantry (the stationary component), it would be appreciated that these may be swapped. That is, in some embodiments, the rod 410 may be fixed in relation to the gantry and the tube may be fixed in relation to the imaging panel 210. In either case, relative movement of the rod and tube leads to the braking forces described above.

### Magnetic Speed control

In addition to or instead of using a pneumatic braking system as described above, some embodiments may use a magnetic braking system to control the speed of the imaging panel as it moves between positions. In one embodiment, a magnetic eddy current brake may be used to brake or decrease the speed of the imaging panel. This may be done by fixing a conductive non-magnetic material such as a copper plate to the imaging panel, and one or more permanent magnets to the gantry. Relative movement of the copper plate in the presence of a magnetic field produced by the one or more magnets will give to eddy currents in the copper plate, which in turn produce magnetic fields that oppose the magnetic fields generated by the permanent magnets. As a result, a drag force is exerted on the copper plate (and in turn the imaging panel) which is slowed. Alternatively, the copper plate (or other conductive non-magnetic material) may be fixed to the gantry and the magnets may be fixed in relation to the imaging panel. In either case, relative movement of the conductive non-magnetic material in the presence of a magnetic field generated by the permanent magnet produces a braking force on the imaging panel.

In an alternative embodiment, the permanent magnet of a magnetic braking system may be replaced with an electromagnet, which may also be used to drive a locking mechanism as described in more detail below.

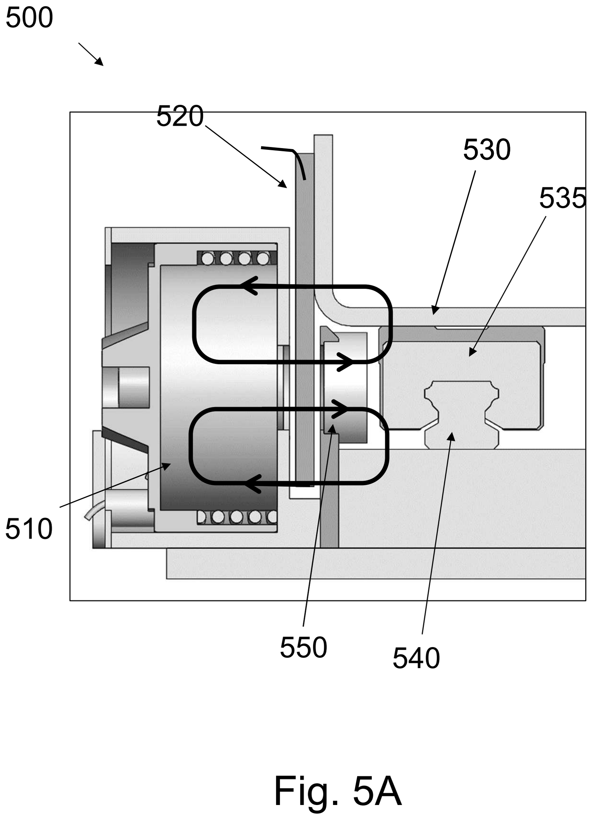

Figure 5A shows a magnetic braking system 500 comprising an electromagnet 510 according to some embodiments of the present disclosure. The electromagnetic braking system 500 comprises a conductive non-magnetic plate 520 (e.g. a copper plate, aluminum plate, or a plate made from any other suitable material that is non-magnetic but that conducts electrical current). The plate 520 is fixed relative to the on-gantry component 530 (e.g. imaging panel 210), which is mounted on a rail 540 that is fixed to the gantry. In this particular example, the on-gantry component comprises a groove track 535 that engages the rail 540 such that the groove track 535 can slide along the rail (in a direction into or out of the plane of the figure). The on-gantry component 530 comprising the plate 620 can therefore travel along the rail, e.g. between two positions (215A and 215B) on the gantry.

In operation, when electrical current is supplied to the electromagnet 510 (e.g. via a controller configured to control supply of the current to the electromagnet), magnetic fields (indicated by the looped arrows in Figure 5a) are generated. The on-gantry component 530 and in particular the plate 520 mounted to the on-gantry component is arranged adjacent to the electromagnet such that when the on-gantry component 530 travels along the rail 540, the plate moves within the magnetic field (indicated by looped arrows in the Figure) generated by the electromagnet 510, thereby causing a drag force *F_{D}* to be exerted on the on-gantry component for the reasons as explained above.

In some embodiments, a controller that controls supply of electrical current to the electromagnetic can be used to control the speed of the on-gantry component 530 as it travels along the rail 540. This may be achieved by adjusting the current supply, which in turn adjusts the strength of the magnetic field and thus the amount of drag force produced in the plate 520. In some embodiments, the braking system 500 further comprises a field strengthening component 550 which may be a permanent magnetic or a ferro/ferrimagnetic material. The field strengthening component is arranged on the opposite side of the plate 520 to the electromagnet 510 and is used to focus magnetic field so that the field strength in the region of the plate 520 is increased. This can improve the braking efficiency (due to the stronger magnetic field seen by the plate 520)

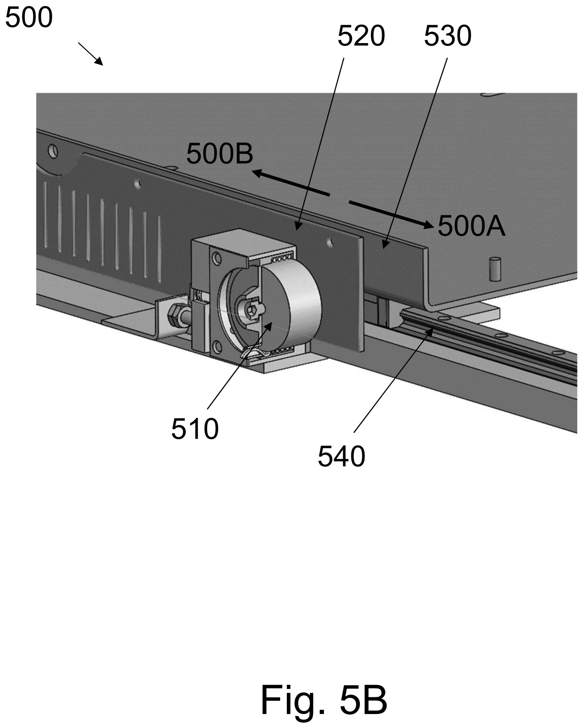

Figure 5B shows a perspective view of the electromagnetic braking system shown in Figure 5A.

Figure 5B includes arrow 500A indicating the direction of movement of the on-gantry component along the rail 540 and arrow 500B indicating the direction of the drag force (opposing movement of the on-gantry component) due to the generation of eddy currents in the plate 520 when the plate passes through a magnetic field generated by the electromagnet 510. As described above, the magnitude of the eddy currents and thus magnitude of the resulting drag force can be controlled by controlling the strength of the magnetic field induced by electromagnet 510. In addition, the magnitude of the drag force can be influenced by the design (e.g. shape and size) of the plate 520.

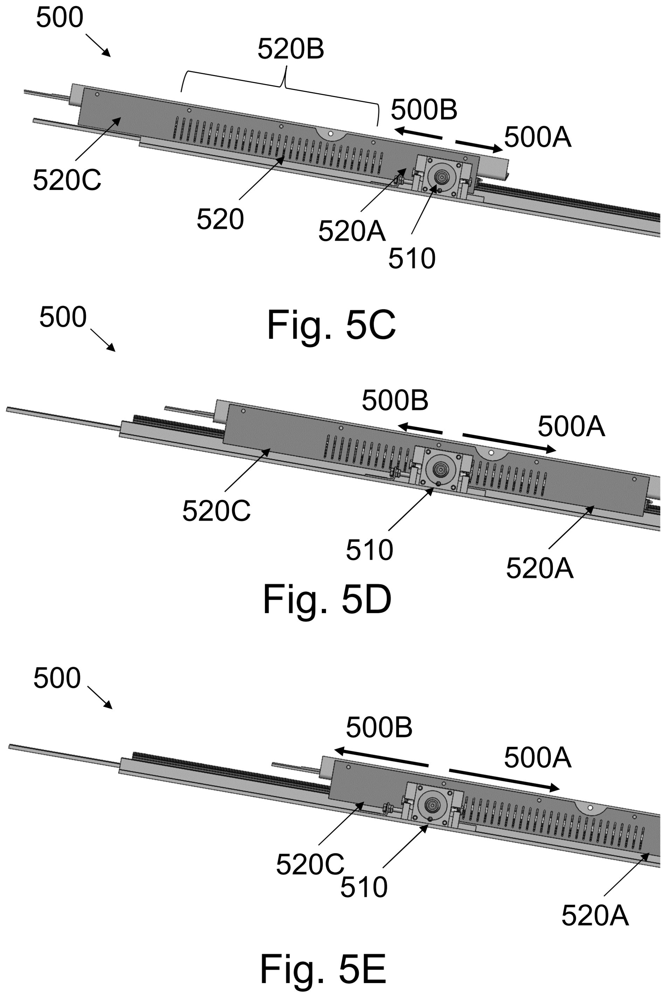

As would be appreciated by the skilled person, the amount of braking achieved by a magnetic braking system can be influenced by the thickness of the plate, the width of the plate, the cross-sectional area of the plate, as well as other features. For example, as can be seen in this figure, the plate 520 comprises a plurality of slots arranged along its length. The function of the slots are described in more detail below with reference to Figure 5C.

Figure 5C shows a side-on side of the electromagnetic braking system 500 shown in Figures 5A and 5B. As can be clearly seen in this Figure, the plate 520 comprises central portion 520B with a slotted design. In particular, the central portion 520B of the plate 520 comprises a plurality of slots or holes arranged along its length. At either end of the central portion 520B, the plate comprises end portions 520A and 520C which do not include slots. The end portions instead are formed of a solid plate of the conductive non-magnetic material (e.g. copper), with a solid cross-section.

The central portion 520B of the plate is designed as a high-speed movement zone relative to the end portions 520A and 520C. The breaks in the plate where the slots/holes are located restrict eddy current generation in the central portion of the plate. In other words, the presence of the slots only permit smaller eddy currents to form in the regions of the plate between the slots. As a result, a smaller drag force is induced when the central portion 520B passes through the magnetic field relative to when either end portion 520A, 520C passes through the magnetic field. This means that the on-gantry component is able to move at a higher speed during a central portion of the range of travel between the first position 215A and the second position 215B, reducing the overall time for the on-gantry component (i.e. the imaging panel) to transition between different positions on the gantry. As the on-gantry component approaches either end position, one of the end portions 520A, 520C of the plate 520 passes through the magnetic field, producing comparatively larger eddy currents in the end portions of the plate and therefore inducing an increased drag force on the on-gantry component, thereby slowing the on-gantry component down before it reaches its final position at either 215A or 215B.

Operation of magnetic braking system 500 is now described with reference to Figures 5C - 5E.

With reference to Figure 5C, the on-gantry component starts at a first position (e.g. position 215A). Optionally, a locking mechanism retaining the on-gantry component at the first position is released prior to or after rotation of the gantry to cause the on-gantry component to travel along the rail(s) under the influence of gravity towards the second position (e.g. position 215B). The on-gantry component then accelerates from an initial velocity 0. At the same time, the drag force induced by eddy current generation in a first end portion 520A of the plate 520 increases proportionally with velocity to control the speed of the on-gantry component as it travels from the first position 215A towards position 215B.

When the on-gantry component has travelled a sufficient distance along the rail (the distance corresponding to the length of the end portion 520A), the on-gantry component reaches a central portion of its travel, corresponding to the central slotted portion 520B of the plate 520. This is shown in Figure 5D. The eddy currents generated as the central portion 520B passes through the magnetic field are smaller than those produced when first end portion 520A passed through the field, meaning the plate experiences less drag force in the slotted central portion compared to the solid end portions. As such, the velocity of travel is higher when the central portion 520B is passing through the magnetic field compared to the velocity of travel when the end portions pass through the magnetic field.

When the on-gantry component approaches the second position 215B (as shown in Figure 5E), the second end portion 620C passes through the magnetic field initially at a high velocity (due to the speed increase in the central portion), which causes an initial high drag force that causes the on-gantry component to decelerate. The on-gantry component then reaches an end stop (e.g. a buffer) that stops the on-gantry component at the second position 215B. Optionally, a locking mechanism may be used to retain the on-gantry component at the second position.

It would be appreciated that other techniques for adjusting the speed during travel of the on-gantry component may be used in addition to or instead of the slotted plate described above. For example, the magnetic field strength could be adjusted during movement (e.g. decreased for a central portion of the range of travel, and then increased as the on-gantry component approaches either end position), and/or the gantry angle could be adjusted (to increase or decrease the gravitational acceleration vector of the on-gantry component acting in the direction of travel). In general, the present disclosure may use any suitable technique to control the speed of the on-gantry component as it travels between two different positions on the gantry.

### Magnetic locking

The electromagnet 510 described above in relation to Figures 5A - 5E can be used to control the speed of the imaging panel as it moves between different positions on the gantry. At the same time, in some embodiments, the same electromagnet 510 can be used as part of a locking mechanism 600 configured to retain the on-gantry component in place at either position (e.g. the first position 215A and second position 215B, or more generally any intermediate position along the rails between 215A and 215B).

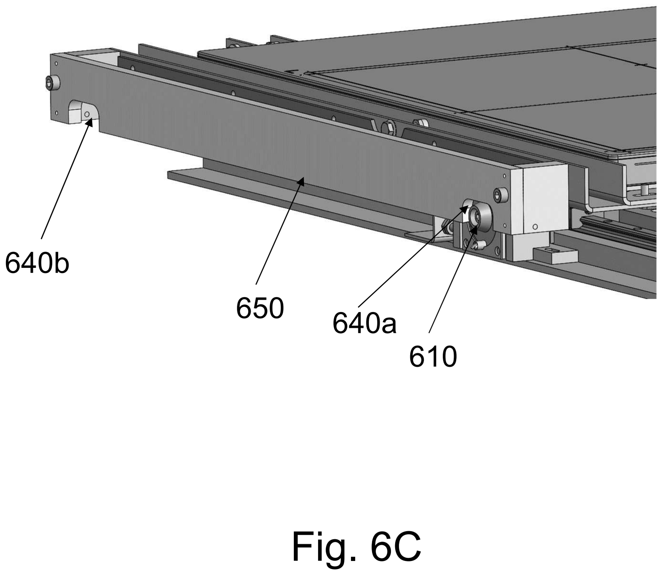

Figures 6A and 6B show a perspective view of locking mechanism 600 comprising an electromagnet 510 in an unlatched position (Figure 6A) and a latched position (Figure 6B). The electromagnet is capable of moving between the latched and unlatched positions. The electromagnet 510 has a locking pin 610 attached thereto and spring 620 that biases the electromagnetic towards the latched position. Arrow 625 indicates the biasing force provided by the spring 620 which drives the electromagnet and locking pin 610 towards the latched position. In the latched position, the locking pin engages with a corresponding portion of the on-gantry component 210 as shown in Figure 6C to retain the on-gantry component in a fixed position (i.e. to prevent the component from moving along the rails).

When the electromagnet 510 is unpowered (i.e. there is no current supply), the spring 620 biases the electromagnet towards the latched position shown in Figure 6B. When current is applied to the electromagnet, a magnetic field is generated. A fixed magnetic component 630 such as permanent magnet or other ferro/ferrimagnetic material is located on the opposite side to the locking pin, i.e. in the opposite direction to the biasing force produced by the spring. The presence of this magnetic component 630 induces a magnetic attraction force in the opposite direction to the spring (indicated by arrow 635) when the electromagnet is powered. If the magnetic field has sufficient strength (i.e. sufficient current supplied to the electromagnet 510), the magnetic attraction force (arrow 635) between the electromagnet 510 and the magnetic component 630 overcomes the spring biasing force (arrow 625) and causes the electromagnet 510 to move from the latched position to the unlatched position, thereby disengaging the locking pin 610 from the on-gantry component and allowing the on-gantry component to freely move along the rails.

As depicted in Figure 6A, wherein the locking mechanism 600 is in the unlatched position, the arrow 635 representing the magnetic force between the electromagnet 510 and the magnetic component 630 is longer than the arrow 625 representing the biasing force from the spring 620, indicating that the magnetic force overcomes the biasing force to drive the locking mechanism to the unlatched position. By contrast, in Figure 6B where the locking mechanism 600 is in the latched position, there is no magnetic force 635 due to the electromagnet being unpowered. Thus, the arrow 625 representing the biasing force from the spring 620 drives the locking mechanism into the latched position.

Figure 6C shows a perspective view of the locking pin 610 engaging with a cutout 640a in a plate 650 attached to the on-gantry component 210, thereby preventing movement of the component along the rails. The on-gantry component 210 is therefore fixed in one position e.g. position 215A by the engagement of the locking pin 610 with the plate 650. The cutout out shape and size corresponds to the shape and size of the locking pin 610 so that the engagement of the locking pin and cutout securely retains the on-gantry component in a fixed position. As can be seen in Figure 6C, plate 650 has cutouts 640a, 640b at either end of the length of the plate, each corresponding to a different one of the end positions 215A or 215B. However, in general, the on-gantry component may be retained by the magnetic locking mechanism in any one of a plurality of different positions between end positions 215A and 215B by means of a corresponding plurality of cutouts along the length of the plate 650.

The electromagnet 600 can therefore function as a combined speed control, braking control and position locking system for a component such as an imaging panel that is configured to move between different positions on a gantry.

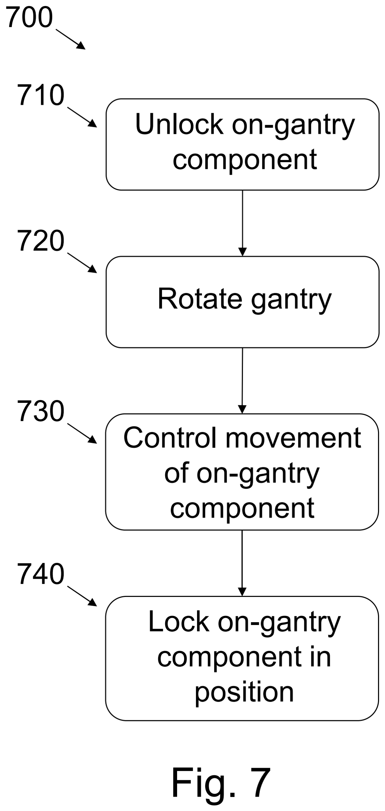

With reference to Figure 7, a method 700 of displacing an on-gantry component of a radiotherapy system is described. At step 710, an on-gantry component 210 (e.g. an imaging panel) is located at a first position (e.g. position 215A) on a gantry. The component 210 is held in place at the first position by a locking mechanism, for example a magnetic latch as described above in relation to Figures 6A -6C. At step 710, the locking mechanism is unlocked such that the on-gantry component is free to slide along one or more rails to which the component is mounted under the influence of an external force. If, at 710, the rails are horizontal, then absent any other external force (e.g. from a human operator), the component will remain in place at the first position.

At step 720, the gantry is rotated to cause the rails to which the component is mounted to be inclined at an oblique angle with respect to the horizontal. As described above in relation to Figures 2 and 3, this causes the component 210 to slide along the rails under the influence of gravity, since a component of the force of gravity acting on the component is parallel to the rail when the rails are inclined. The rotation of the gantry at step 720 therefore causes the component to start moving along the rails away from the first position 215A and towards a second position 215B.

At step 730, whilst the component is moving along the rails under the influence of gravity, the speed of movement of the component may be controlled by using one or more speed control mechanisms or braking systems. In an example, a pneumatic braking system (such as one described above in relation to Figures 4A - 4D) may be used to control and limit the speed of the component as it moves along the rails.. In other examples, a magnetic braking system (such as one described above in relation to Figures 5A - 5E) may be used to control and limit the speed of the component as it moves along the rails. In other examples, the speed of the on-gantry component may be controlled additionally or alternatively using the rotation of the gantry itself. That is,

At step 740, when the component reaches the second position 215B, a locking mechanism (such as a latching mechanism) locks the component in position at the second position, such that further rotation of the gantry in either direction will not cause further movement of the component along the rails.

It would be appreciated that method 700 may be repeated any number of times, starting from either of the first position 215A and second position 215B and ending up at the opposite position. As such, a method of generally moving an on-gantry component between two positions on a gantry of a radiotherapy system is provided.

### Counter balance system

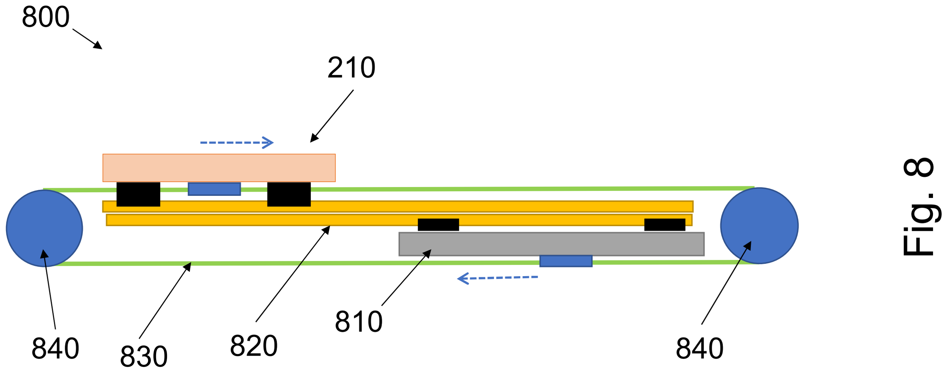

Figure 8 illustrates a counterbalance system 800 according to some embodiments of the present disclosure. The counter balance system 800 comprises a counterbalance weight 810 attached to a rail or track system 820 with a synchronizing belt, wire, or chain 830 and pulley system 840 that couples movement of the counter balance weight 810 to the on-gantry component (e.g. imaging panel 210).

In more detail, the on-gantry component 210 is coupled to the counterbalance weight 810 via the synchronizing belt 830 such that movement of the on-gantry component (e.g. under the influence of gravity) in one direction causes an equal and opposite movement of the counterbalance weight. As shown in Figure 8, when the on-gantry component moves to the right (e.g. from a first position 215A to a second position 215B under the influence of gravity), the counterbalance weight 810 correspondingly moves an equal amount to the left.

The counterbalance 810 is of a similar mass to the on-gantry component, meaning that there is very little shift in the center of mass of the overall system 800 when the on-gantry component moves between positions on the gantry. The counterbalance weight 810 is however of a smaller mass than the on-gantry component, so as not to prevent free movement under gravity of the of on-gantry component when the gantry rotates (as described above). In particular, the mass of the counterbalance weight 810 must be sufficiently less than the mass of the on-gantry component such that the difference in weight remains greater than the sum of resistive forces (including friction in the rail or track system 820 and the synchronizing system 830, 840) that oppose movement of the on-gantry component. In other words, the counterbalance weight 810 should not be so large that it prevents the on-gantry component from freely moving under the influence of gravity along the track/rails to which it is mounted.

The use of a counterbalance system 800 may be desirable since it can decrease the dynamic and static unbalance of a rotating system. If the moving parts are heavy and/or the angular speeds of the gantry are high, the counterbalance system may be preferable since it reduces the shift in the centre of mass of the gantry when the on-gantry component moves position. The static unbalance that a moving mass cause will affect the drive of the gantry and may require an adapted driving and braking in order to safely operate (rotate) the gantry. However, using a counterbalance system as described above, the need for such a specially adapted drive/brake system may be avoided.

In some embodiments, the counterbalance weight is made of a radiation attenuation material and function as a radiotherapy beam stopper/shield. In more detail, in some embodiments, the on-gantry component is an MV imaging panel. During radiotherapy treatment, the panel itself may be moved out the path of a radiotherapy beam to protect the components of the imaging panel (e.g. by moving the panel from one position, e.g. 215A, to another position 215B that is out of the path of the beam). As a result, the counterbalance weight may be moved in the opposite direction and into the beam path. The counterbalance weight can therefore function as a beam stopper as well as a counterbalance, since in any case the counterbalance will necessarily be in the path of the radiotherapy beam. This reduces or potentially entirely removes the need for a separate dedicated beam stopper, since this function is already partially or fully fulfilled by the counterweight balance 810.

### Radiotherapy System

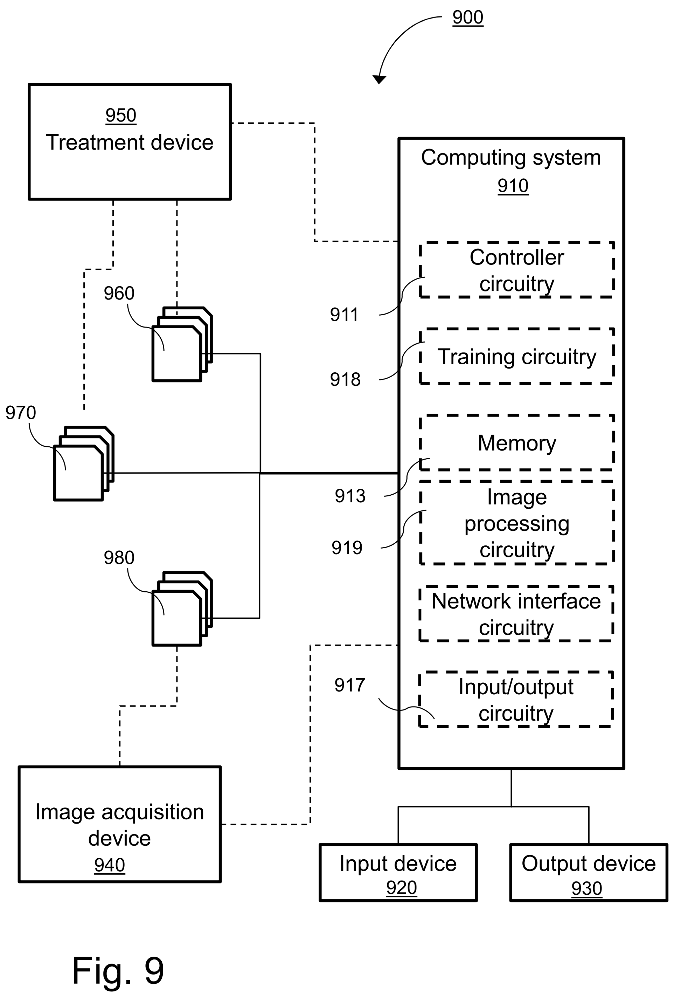

Figure 9 illustrates a block diagram of one implementation of a radiotherapy system 900. The radiotherapy system 900 comprises a computing system 910 within which a set of instructions, for causing the computing system 910 to perform any one or more of the methods discussed herein, may be executed.

The computing system 910 shall be taken to include any number or collection of machines, e.g. computing device(s), that individually or jointly execute a set (or multiple sets) of instructions to perform any one or more of the methods discussed herein. That is, hardware and/or software may be provided in a single computing device, or distributed across a plurality of computing devices in the computing system. In some implementations, one or more elements of the computing system may be connected (e.g., networked) to other machines, for example in a Local Area Network (LAN), an intranet, an extranet, or the Internet. One or more elements of the computing system may operate in the capacity of a server or a client machine in a client-server network environment, or as a peer machine in a peer-to-peer (or distributed) network environment. One or more elements of the computing system may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine.

The computing system 910 includes controller circuitry 911 and a memory 913 (e.g., read-only memory (ROM), flash memory, dynamic random access memory (DRAM) such as synchronous DRAM (SDRAM) or Rambus DRAM (RDRAM), etc.). The memory 913 may comprise a static memory (e.g., flash memory, static random access memory (SRAM), etc.), and/or a secondary memory (e.g., a data storage device), which communicate with each other via a bus (not shown).

Controller circuitry 911 represents one or more general-purpose processors such as a microprocessor, central processing unit, accelerated processing units, or the like. More particularly, the controller circuitry 911 may comprise a complex instruction set computing (CISC) microprocessor, reduced instruction set computing (RISC) microprocessor, very long instruction word (VLIW) microprocessor, processor implementing other instruction sets, or processors implementing a combination of instruction sets. Controller circuitry 911 may also include one or more special-purpose processing devices such as an application specific integrated circuit (ASIC), a field programmable gate array (FPGA), a digital signal processor (DSP), network processor, or the like. One or more processors of the controller circuitry may have a multicore design. Controller circuitry 911 is configured to execute the processing logic for performing the operations and steps discussed herein.

The computing system 910 may further include a network interface circuitry 915. The computing system 910 may be communicatively coupled to an input device 920 and/or an output device 930, via input/output circuitry 917. In some implementations, the input device 920 and/or the output device 930 may be elements of the computing system 910. The input device 920 may include an alphanumeric input device (e.g., a keyboard or touchscreen), a cursor control device (e.g., a mouse or touchscreen), an audio device such as a microphone, and/or a haptic input device. The output device 930 may include an audio device such as a speaker, a video display unit (e.g., a liquid crystal display (LCD) or a cathode ray tube (CRT)), and/or a haptic output device. In some implementations, the input device 920 and the output device 930 may be provided as a single device, or as separate devices.

In some implementations, the computing system 910 may comprise image processing circuitry 919. Image processing circuitry 919 may be configured to process image data 980 (e.g. images, or imaging data), such as medical images obtained from one or more imaging data sources, a treatment device 950 and/or an image acquisition device 940. Image processing circuitry 919 may be configured to process, or pre-process, image data. For example, image processing circuitry 919 may convert received image data into a particular format, size, resolution or the like. In some implementations, image processing circuitry 919 may be combined with controller circuitry 911.

In some implementations, the radiotherapy system 900 may further comprise an image acquisition device 940 and/or a treatment device 950, such as those disclosed herein in the examples of Figure 1. The image acquisition device 940 and the treatment device 950 may be provided as a single device. In some implementations, treatment device 950 is configured to perform imaging, for example in addition to providing treatment and/or during treatment. The treatment device 950 comprises the main radiation delivery components of the radiotherapy system, such as the Linac, beam guides and beam shaping systems including a multi-leaf collimator.

Image acquisition device 940 may be configured to perform positron emission tomography (PET), computed tomography (CT), magnetic resonance imaging (MRI), or any other suitable imaging technique. Image acquisition device 940 may be configured to output image data 980, which may be accessed by computing system 910. Treatment device 950 may be configured to output treatment data 960, which may be accessed by computing system 910.

Computing system 910 may be configured to access or obtain treatment data 960, planning data 970 and/or image data 980. Treatment data 960 may be obtained from an internal data source (e.g. from memory 913) or from an external data source, such as treatment device 950 or an external database. Planning data 970 may be obtained from memory 913 and/or from an external source, such as a planning database. Planning data 970 may comprise information obtained from one or more of the image acquisition device 940 and the treatment device 950.

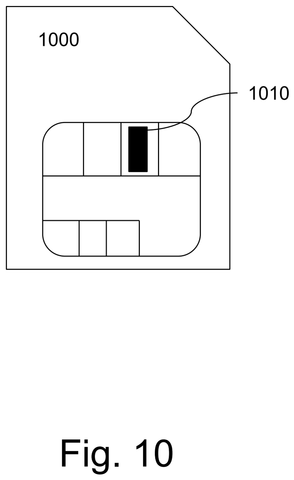

The various methods described above may be implemented by a computer program. The computer program may include computer code (e.g. instructions) 1010 arranged to instruct a computer to perform the functions of one or more of the various methods described above. The steps of the methods described above may be performed in any suitable order. For example, step 710 of method 700 may be performed simultaneously or substantially simultaneously with step 720 . The computer program and/or the code 1010 for performing such methods may be provided to an apparatus, such as a computer, on one or more computer readable media or, more generally, a computer program product 100)), depicted in Figure 10. The computer readable media may be transitory or non-transitory. The one or more computer readable media 1000 could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD. The instructions YY10 may also reside, completely or at least partially, within the memory 913 and/or within the controller circuitry 911 during execution thereof by the computing system 910, the memory 913 and the controller circuitry 911 also constituting computer-readable storage media.

In an implementation, the modules, components and other features described herein can be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may comprise a special-purpose processor, such as an FPGA or an ASIC. A hardware component may also include programmable logic or circuitry that is temporarily configured by software to perform certain operations.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

Unless specifically stated otherwise, as apparent from the following discussion, it is appreciated that throughout the description, discussions utilizing terms such as "receiving", "determining", "comparing ", "enabling", "maintaining," "identifying", or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

It is to be understood that the above description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described, but can be practiced with modification and alteration within the spirit and scope of the appended claims. Accordingly, the specification and drawings are to be regarded in an illustrative sense rather than a restrictive sense. The scope of the disclosure should, therefore, be determined with reference to the appended claims, along with the full scope of equivalents to which such claims are entitled.

## Claims

1. A radiotherapy system comprising:
a rotatable gantry configured to rotate about a rotation axis;
an on-gantry component mounted to the gantry, wherein the on-gantry component is configured to travel between at least a first position and a second position on the gantry, and wherein the on-gantry component is mounted to the gantry such that when the gantry is rotated in a first direction about the rotation axis, the on-gantry component is capable of travelling from the first position to the second position under the influence of gravity.

2. The system of claim 1, further comprising a first locking mechanism configured to engage and disengage with a portion of the on-gantry component to retain the on-gantry component at the first position, wherein the first locking mechanism prevents the on-gantry component from travelling to the second position when the first locking mechanism is engaged with the on-gantry component.

3. The system of any preceding claim, wherein the on-gantry component is further capable of travelling from the second position to the first position under the influence of gravity when the gantry is rotated in a second direction about the rotation axis, the second direction being opposite to the first direction.

4. The system of any preceding claim, further comprising a second locking mechanism configured to engage and disengage with a portion of the on-gantry component to retain the on-gantry component at the second position, wherein the second locking mechanism prevents the on-gantry component from travelling to the first position when the second locking mechanism is engaged with the on-gantry component.

5. The system of any preceding claim, wherein the on-gantry component is configured to move between the first and the second positions without requiring a drive force from a dedicated drive system connected to the on-gantry component.

6. The system of any preceding claim, wherein the on-gantry component is slidably mounted to one or more rails extending generally between the first and second positions on the gantry, such that a gravitational force acting on the on-gantry component is capable of causing the on-gantry component to slide along the rails, optionally wherein the on-gantry component is capable of travelling, under the influence of gravity, along the rails, when the gravitational force acting on the on-gantry component is at least partially in the same direction as the direction of travel along the rails.

7. The system of any preceding claim, further comprising a motion control means configured to control the speed of the on-gantry component during travel between the first and second position.

8. The system of claim 7, wherein the motion control means comprises a braking system configured to limit the speed of the on-gantry component during at least a portion of the travel between the first and second position, optionally wherein the braking system comprises a pneumatic braking system comprising a rod located inside and configured to move within a tube, wherein one of the rod and the tube is fixed relative to the on-gantry component and wherein the other one of the rod and the tube is fixed relative to the gantry, such that movement of the on-gantry component between the first and second positions on the gantry causes movement of the rod relative to the tube, and wherein the rod and tube arrangement is configured to apply a braking force to the on-gantry component when the on-gantry component travels between the first and second positions, and optionally wherein the rod is connected to either the on-gantry component or the gantry via a connecting portion extending through an open end of the tube, and wherein the opposite end of the tube is closed such that movement of the rod within the tube causes expansion and compression of air located in the tube between the rod and the closed end of the tube, and wherein said expansion or compression of air exerts a force on the rod that opposes the movement of the rod relative to the tube, and optionally wherein the closed end of the tube comprises a valve configured to control the flow of air into and out of the tube, optionally wherein the tube comprises a central portion and end portions located at opposite ends of the central portion, wherein the internal diameter of the central portion is larger than the internal diameter of either end portion.

9. The system of any of claim 8, wherein the pneumatic braking system comprises a first rod configured to move within a first tube, the first rod and tube configured to apply a braking force to the on-gantry component when the on-gantry component travels towards the first position, and a second rod configured to move within a second tube, the second rod and tube configured to apply a braking force to the on-gantry component when the on-gantry component travels towards the second position.

10. The system of claim 8, wherein the braking system comprises a magnetic braking system comprising one or more magnets arranged adjacent to one or more conductive non-magnetic elements, wherein either the one or more magnets or the one or more conductive non-magnetic elements are fixed relative to the on-gantry component, and wherein the other of the one or more magnets and the one or more conductive non-magnetic elements is fixed relative to the gantry, such that movement of the on-gantry component between the first and second positions causes relative movement between the one or more magnets and one or more conductive non-magnetic elements, optionally wherein the one or more magnets comprises an electromagnet, and wherein the system further comprises a controller configured to control power supply to the electromagnet.

11. The system of claim 10, wherein the one or more conductive non-magnetic elements comprises an elongate metallic plate with a length that substantially corresponds to the distance between the first and second positions on the gantry, optionally wherein the elongate metallic plate comprises a central portion and end portions located at either end of the central portion, wherein the plate comprises a plurality of holes or slots arranged along the length of the central portion.

12. The system of claim 2 or 4, wherein the first and second locking mechanism comprises an electromagnetic latch configured to move between a latched position and an unlatched position to retain the on-gantry component at either the first or second position, wherein the electromagnetic latch comprises:
an electromagnet and a locking pin attached thereto;
a spring that biases the electromagnetic latch towards the latched position; and
a magnetic component arranged on an opposite side of the electromagnet to the locking pin,
optionally wherein, when in the latched position, the locking pin is configured to engage a portion of the on-gantry component to retain the on-gantry component at either the first or second position, and wherein the electromagnetic latch is arranged such that power supplied to the electromagnet causes a magnetic attraction force between the electromagnet and the magnetic component to drive the electromagnetic latch to an unlatched position whereby the locking pin is disengaged from the on-gantry component.

13. The system of any preceding claim further comprising a counterbalance system configured offset the change in weight distribution on the gantry when the on-gantry component travels between the first and second position, wherein the counterbalance system comprises a counterbalance weight configured to move in generally the opposite direction to the on-gantry component as the on-gantry component travels between the first and second positions on the gantry, optionally wherein the counterbalance weight is connected to the on-gantry component via one or more of: a synchronizing belt; a wire; a chain pulley system; gears; and link arm mechanics, such that movement of the counterbalance weight is synchronized with movement of the on-gantry component.

14. The system of claim 22 or 23, wherein the counterbalance weight comprises a radiation attenuating material.

15. A method for displacing an on-gantry component of a radiotherapy system of any preceding claim, the method comprising:
when the on-gantry component is retained at the first position on the gantry by a first locking mechanism, unlocking the first locking mechanism such that the on-gantry component is free to move towards the second position on the gantry;
causing the gantry to rotate in a first direction about the rotation axis such that a gravitational force acting on the on-gantry component causes the on-gantry component to travel from the first position to the second position under the influence of gravity;
controlling the speed of the on-gantry component whilst the on-gantry component is travelling from the first position to the second position, wherein controlling the speed of the on-gantry component comprises:
using a pneumatic or magnetic braking system to limit the speed of the on-gantry component; or
rotating the gantry whilst the on-gantry component is travelling between the first and second position to adjust a gravitational acceleration vector acting on the on-gantry component; and
when the on-gantry component reaches the second position, retaining the on-gantry component at the second position using a second locking mechanism such that further rotation of the gantry will not cause further movement of the on-gantry component.
